# EUROPÄISCHE PATENTSCHRIFT

(11) **EP 1 389 242 B1**
(45) Veröffentlichungstag und Bekanntmachung des Hinweises auf die Patenterteilung: **18.04.2007**
(21) Anmeldenummer: 02732730.3
(22) Anmeldetag: 21.05.2002
(51) Int. Cl.: C12Q 1/68, C12N 15/11

(54) **NACHWEIS UND DIFFERENZIERUNG VON MIKROORGANISMEN DER SPEZIES YERSINIA PESTIS/YERSINIA PSEUDOTUBERCULOSIS**
DETECTING MICROORGANISMS OF THE YERSINIA PESTIS/YERSINIA PSEUDOTUBERCULOSIS SPECIES AND/OR DIFFERENTIATING BETWEEN YERSINIA PESTIS AND YERSINIA PSEUDOTUBERCULOSIS
DETECTION DE MICROORGANISMES DE L'ESPECE YERSINIA PESTIS/YERSINIA PSEUDOTUBERCULOSIS ET/OU DIFFERENCIATION ENTRE YERSINIA PESTIS ET YERSINIA PSEUDOTUBERCULOSIS

(30) Priorität: 18.05.2001 DE 10124342
(43) Veröffentlichungstag der Anmeldung: 18.02.2004
(73) Patentinhaber: Biotecon Diagnostics GmbH, 10589 Berlin (DE)
(72) Erfinder: GRABOWSKI, Reiner, 79576 Weil am Rhein (DE); KOHLHAUSSEN, Simone, 14480 Potsdam (DE); BERGHOF, Kornelia, 12355 Berlin (DE)
(74) Vertreter: Grünecker, Kinkeldey, Stockmair & Schwanhäusser Anwaltssozietät
(86) Internationale Anmeldenummer: PCT/EP2002/005580
(87) Internationale Veröffentlichungsnummer: WO 2002/095066

(56) Entgegenhaltungen:
- WO-A-00/69897
- WO-A-95/11996
- WO-A-96/08582
- WO-A-97/41257
- WO-A-99/22023
- DE-A- 19 945 916
- US-A- 5 620 847
- US-A- 6 001 564
- NORKINA OLGA V ET AL: "Development of a diagnostic test for Yersinia pestis by the polymerase chain reaction." JOURNAL OF APPLIED BACTERIOLOGY, Bd. 76, Nr. 3, 1994, Seiten 240-245, XP009003977 ISSN: 0021-8847
- TREBESIUS K ET AL: "Development of rRNA targeted PCR and in situ hybridization with fluorescently labelled oligonucleotides for the detection of Yersinia species" JOURNAL OF CLINICAL MICROBIOLOGY, WASHINGTON, DC, US, Bd. 36, Nr. 9, September 1998 (1998-09), Seiten 2557-2564, XP002204235 ISSN: 0095-1137
- WOUDENBERG T. M. ET AL.: "QUANTITATIVE PCR BY REAL TIME DETECTION" PROCEEDINGS OF THE SPIE, Bd. 2680, 1996, Seiten 306-315, XP000197422
- NEUBAUER H. ET AL.: "A combination of different polymerase chain reaction (PCR) assays for the presumptive identification of Yersinia pestis." JOURNAL OF VETERINARY MEDICINE SERIES B, Bd. 47, Nr. 8, Oktober 2000 (2000-10), Seiten 573-580, XP009003968 ISSN: 0931-1793 in der Anmeldung erwähnt
- DATABASE GENSEQ [Online] 7. Oktober 1999 (1999-10-07) GRIFFAIS R.: "PCR primer used to amplify an ORF of Chlamydia trachomatis." Database accession no. AAZ05841 XP002227316
- GIBELLO A. ET AL.: "Development of a PCR assay for detection of Yersinia ruckeri in tissues of inoculated and naturally infected trout." APPLIED AND ENVIRONMENTAL MICROBIOLOGY, Bd. 65, Nr. 1, Januar 1999 (1999-01), Seiten 346-350, XP002227315 ISSN: 0099-2240
- SCHEU P. ET AL.: "RAPID DETECTION OF LISTERIA MONOCYTOGENES BY PCR-ELISA" LETTERS IN APPLIED MICROBIOLOGY, Bd. 29, Nr. 4, Dezember 1999 (1999-12), Seiten 416-420, XP000892485

## Beschreibung

Die vorliegende Erfindung betrifft ein Verfahren zum Nachweis von Mikroorganismen der Spezies *Yersinia pestis*/*Yersinia pseudotuberculosis* und/oder zur Differenzierung zwischen *Yersinia pestis* und *Yersinia pseudotuberculosis.*

Die Pest, verursacht durch *Yersinia pestis,* stellt auch heute noch eine Gefahr für die öffentliche Gesundheit dar. Es bestehen weltweit natürliche Reservoire in vielen Ländern Afrikas, Amerikas und Asiens. In dem Zeitraum von 1990 bis 1997 ist die Zahl der registrierten Pestfälle von 1257 auf 5419 gestiegen (WHO, 1999). Die Zunahme an internationalen Reisen bringt ein zusätzliches Risiko zur Verbreitung der Krankheit, ausgehend von endemischen Regionen, durch infizierte Reisende mit sich. Eine präzise und schnelle Diagnose der Pest würde ein schnelles Eingreifen im Falle eines Pestausbruchs ermöglichen. Dabei ist die Kenntnis der gerade zirkulierenden Stämme in jedem Herd die Voraussetzung für die Ergreifung wirksamer Maßnahmen (Leal *et al.,* 1999, *Rev. Inst. Med. Trop. Sao Paulo* 41, 339-342).

Die Gattung *Yersinia* gehört zur Familie der Enterobacteriaceae. Zu ihr zählen derzeit 11 Spezies, davon sind die Spezies - *Yersinia- pestis*/*Yersinia pseudotuberculosis* und *Yersinia enterocolitica* humanpathogen. *Y. pestis* und *Y. pseudotuberculosis* weisen eine 90%ige DNA Sequenzhomologie auf, gegenüber *Y. enterocolitica* weisen beide jedoch nur eine 50%ige Homologie auf. *Y. pestis* und *Y. pseudotuberculosis* gelten daher als Pathovare einer einzigen Spezies. Trotz der hohen Sequenzhomologie gibt es jedoch fundamentale Unterschiede zwischen *Y. pestis* und *Y. pseudotuberculosis,* wie den Modus der Übertragung sowie die unterschiedliche Letalitätsrate der ausgelösten Erkrankungen und, daraus resultierend, eine unterschiedliche historische Bedeutung der Erreger. Dennoch wurden die Pathovare nicht als Subspezies reklassifiziert (Achtman et al., 1999, *Proc.Natl.Acad.Sci.U.S.A.* 96, 14043-14048).

*Yersinia pestis* (syn. *Pasteurella pestis*), benannt nach dem französischen Bakteriologen A.J.E. Yersin, der das Bakterium 1894 erstmals isolierte, ist ein unbegeißeltes, sporenloses, pleomorphes, Gram-negatives Bakterium. Es ist der Erreger der Bubonen- bzw. Beulenpest und kann durch Flöhe von infizierten Ratten und anderen Nagetieren (Feldmäuse, Ziesel, Tarbaganen, Erdhörnchen) auf den Menschen übertragen werden.
Früher war es weltweit verbreitet, heute kommt es jedoch nur noch in einzelnen enzootischen und epizootischen Herden vor (Bergwald- und Savannenregionen in Amerika, Zentral-, Ost- und Südafrika, Madagaskar, Zentral- und Südostasien). *Yersinia pestis* ist in der Lage, auch monatelang in Sputum, Kot, Eiter oder in Ektoparasiten eingetrocknet zu überleben, was die spontanen Restvorkommen (auch bei Affen, Schleichkatzen, Kamelen und Schafen) erklärt. Die drei bekannten Biovare (bv antiqua, bv medievalis, bv orientalis bzw. oceanic) unterscheiden sich in ihrem geographischen Verbreitungsspektrum und Reservoir. Vollständig pathogene *Y. pestis* Isolate sind charakterisiert durch die Präsenz von drei unterschiedlichen Plasmiden. Das für *Y. pestis* spezifische 9,5 kb Plasmid pPIa (auch genannt pPCP1 oder pPst) trägt ein Plasminogen-Aktivator/Koagulase-Gen (pla-Gen) und ein Pesticin-Gen (Neubauer *et al.,* 2000, *J. Vet. Med.,* B47, 573-580). Das zweite Pest-spezifische Plasmid pFra (bzw. pMT1) hat eine Größe von 110 kb. Dieses kodiert u.a. für das F1 Kapsel-Antigen. Das dritte Plasmid pYV (bzw. pCD1 oder pCad) ist 70 kb groß und kommt bei allen pathogenen Isolaten der humanpathogenen Spezies *Y. pestis*/*Y pseudotuberculosis* und *Y. enterocolitica* vor. Die dazu bereits bekannten Gensequenzen sind hinterlegt in der GenBank Sequence Database des National Centre for Biotechnology Information (NCBl) (pla-Gen (im Plasmid pPCP1): Accessionnumber AF053945-1; F1-Antigen: Accessionnumber X61996; 16S ribosomale RNA: Accessionnumber L37604). Es sind vier klinische Formen der Pest beschrieben (Pestsepsis, Beulen-, Lungen-, abortive Pest). Unbehandelt liegt die Letalität der Beulenpest bei ca. 30 - 40%. Deshalb begründet bereits der Verdachtsfall den sofortigen Beginn einer antibiotischen Therapie.

*Yersinia pseudotuberculosis* ist ein bei kleinen Nagem, Katzen und Vögeln weit verbreitetes Pathovar, das nur geringe humanmedizinische Bedeutung hat. Es handelt sich um pleomorphe, peritrich begeißelte, bewegliche Kurzstäbchen. Menschliche Infektionen nehmen immer ihren Ausgang von erkrankten Tieren. Die Übertragung erfolgt wahrscheinlich direkt durch Schmierinfektion oder eventuell indirekt über kontaminierte Lebensmittel. Dabei werden die Erreger oral aufgenommen. Die Pseudotuberkulose des Menschen tritt nur sporadisch und sehr selten auf. Der im allgemeinen gutartige Verlauf der Erkrankung erfordert keine spezifische Therapie.

Die Detektion und Identifikation von *Yersinia pestis* mit konventionellen mikrobiologischen Verfahren ist zum Einen sehr zeitaufwendig und zudem, auf Grund der hohen Pathogenität von *Y. pestis* (L3), mit Gefahren für das Laborpersonal des Analyselabors verbunden. Zum Anderen erhöht das vermehrte Auftreten von Resistenzen gegen bestimmte Antibiotika bei humanpathogenen Erregern die Notwendigkeit einer spezifisch auf den jeweiligen Erreger angepaßten Therapie und somit auch die Notwendigkeit einer schnellen und eindeutigen Identifikation des Erregers. Deshalb besteht ein erheblicher Bedarf an Schnellnachweismethoden für humanpathogene Erreger wie *Y. pestis.*

Für den routinemäßigen Einsatz zur Detektion von humanpathogenen Mikroorganismen sind in den letzten Jahren eine Reihe neuer Methoden entwickelt worden. Hierzu zählen z.B..immunologische Verfahren, die auf dem Einsatz polyvalenter oder monoklonaler Antikörper beruhen, sowie Verfahren, bei denen Nukleinsäure-Sonden zum Nachweis Erreger-spezifischer Nukleinsäuren mittels Hybridisierung eingesetzt werden. Als weitere Methoden werden diejenigen Verfahren beschrieben, die auf einer spezifischen Nukleinsäure-Amplifikation basieren, mit oder ohne anschließender Bestätigungsreaktion durch Nukleinsäure-Hybridisierung. Geeignete Verfahren zur Amplifikation von Nukleinsäuren sind z.B. die Polymerase-Kettenreaktion (polymerase chain reaction, PCR) (US Patente 4683195; 4683202; 4965118), die Ligase-Kettenreaktion (WO 89109835), die "self-sustained sequence replication" (EP 329822), das "transcription based amplification system" (EP 310229), das Qß RNA-Repükase-System (US 4957858) oder eine isotherme Nukleinsäureamplifikation. Die genannten auf Nukleinsäure basierenden Verfahren sind so sensitiv, dass im Gegensatz zu konventionellen mikrobiologischen Verfahren eine langwierige Anreicherung des nachzuweisenden Mikroorganismus aus der zu untersuchenden Probe nicht notwendig ist.

Allerdings ist eine Detektion der Erreger (DNA) über PCR und nachfolgende Visualisierung der PCR Produkte über Gelelektrophorese allein oft nicht ausreichend, da damit kein spezifischer Nachweis der Amplifikate durchgeführt wird. Eine Analyse über Gelelektrophorese zeigt lediglich die Größe und Menge des gebildeten Amplifikates an, nicht aber die Sequenz ("identität") des Amplifikationsproduktes.

*Für Yersinia pestis* sind bereits einige spezifische DNA-Sequenzen bekannt 1993 wurde bereits eine erste "nested" PCR von Campbell *et al.* (1993, *J*. *Clin. Microbiol.* 31, 758-759) aus dem Plasminogen-Aktivator-Gen für den Nachweis von *Yersinia pestis* etabliert, wenig später im selben Jahr publizierten Hinnebusch und Schwan (1993, *J. Clin. _{M}fcrobiol.* 31, 1511-1514) für denselben Genbereich auch einen PCR-gestützien Nachweis. Die Autoren amplifizierten nur einen Genbereich, was zu falsch negativen Resultaten führen kann, wenn dieser Genbereich fehlt oder nur teilweise vorhanden ist Tsukano *et al.* (1996, *Microbiol. Immunol.* 40, 773-775) verwendet ein Multiplex-PCR-System zum Nachweis von *Y*. *pestis.* Diese Nachweissysteme haben den Nachteil, dass sie ausschließlich auf *Yersinia* Plasmide als diagnostische Zielmoleküle zurückgreifen. Plasmide können jedoch leicht verloren gehen oder auf andere Bakterien, Insbesondere Enterobakterien, übertragen werden (Allen *et al.,* 1987, *Contr. Microbiol. Immunol.,* 9*,* 332-341). Der Nachweis von Plasmiden allein gewährleistet also keine Sicherheit bei der Identifizierung von *Yersinia pestis.*

In Trebesius et al, *J*. *of Clinic. Microbiol.,* 9 (1998) 2557-2564 wird der Nachweis von *Yersinia-Arten* mit Hilfe von 16S und 23S rDNA-Genen beschrieben. Eine Differenzierung der Arten von *Y. pestis* und *Y. pseudotuberculosis* wird dabei nicht erreicht.

Ein Nachteil aller bekannten Verfahren ist weiterhin, dass bei Einsatz von Nukleinsäuresequenzen als Primer bei der Polymerase-Kettenreaktion falsch negative Resultate auftreten können. Dies wurde bereits für andere Systeme zum Nachweis von Krankheitserregem wie *bovine leukemia virus* (BLV-PCR) und *feline infectious peritonitis virus* (FIPV-RT-PCR) gezeigt (Ballagi-Pordany *et al.* 1996, *Mol. Cell Probes* 10, 159-164). Der Nachweis von mehreren verschiedenen Nukleinsäuresequenzen, insbesondere von "Virulenz-Markern" wurde auch von Leal *et al.* (1999, *Rev. inst. Med*. *Trop. Sao Paulo* 41, 339-342) aufgezeigt Da das Nachweissystem jedoch auch auf Plasmide zurückgreift, ist seine Spezifität nicht gewährleistet Außerdem genügt die Sensitivität dieses PCR-Systems nicht den Anforderungen eines industriellen Qualitätsmanagements.

Auch Neubauer *et al.* (2000, *J*. *Vet. Med. B. Infect. Dis. Vet. Public Health* 47, 573-580) kombinierten verschiedene "Virulenz-Marker". Zusätzlich wird als "chromosomaler Marker" ein Abschnitt der 16S rDNA nachgewiesen. Die vier Zieigenabschnitte werden hier nicht durch Multiplex-PCR, sondern über separate PCR-Ansätze nachgewiesen. Die Prüfung der Funktionalität dieses *Yersinia pestis* PCR-Systems (Neubauer *et al.)* zeigt jedoch, dass Primer angegeben sind, die nur zum Teil (F1-Antigen) bzw. gar nicht (16 S) die Zielsequenz amplifizieren. Darüber hinaus wurde bei diesen und den zuletzt genannten Nachweissystemen für *Y. pestis* auf die Implementierung einer Spezifitätskontrolle verzichtet. Daher sind auch hier falsch positive bzw. falsch negative Resultate möglich.

Der vorliegenden Erfindung liegt daher die Aufgabe zugrunde, sichere Nachweis- und/oder Differenzierungsverfahren für Mikroorganismen der Spezies *Yersinia pestis*/*yersinia pseudotuberculosis* zur Verfügung zu stellen.

Diese Aufgabe wird erfindungsgemäß gelöst durch ein Verfahren zum Nachweis von Mikroorganismen der Spezies *Yersinla pesüs*/*Yersinia pseudotuberculosls* und zur Differenzierung zwischen den Pathovaren *Yersinla pestis* und *Yersinia pseudotuberculosis* In einer Probe, wobei das Verfahren folgende Schritte umfaßt:
(a) Inkontaktbringen der Probe mit einer Kombination aus mindestens zwei Primerpaaren die mit einem in der Spezies *Yersinia pestis*/*Yersinia pseudotuberculosis* konservierten Bereich ihres mikrobiellen Genoms oder eines Teils desselben hybridisieren, wobei ein erstes Primerpaar mit einem konservierten Bereich eines bakteriellen 16S rDNA-Genes oder eines Teils desselben hybridisiert und ein zweites Primerpaar mit einem konservierten Bereich der Plasmide pPla, pFra oder pYV aus *Yersinia pestis* oder eines Teils derselben hybridisiert;
(b) Amplifikation der mikrobiellen Genome oder eines Teils derselben zur Erzeugung von mindestens zwei Amplifikationsfragmenten;
(c) Inkontaktbringen der in Schritt (b) erhaltenen Amplifikationsfragmente mit mindestens einer für *Yersinia pestis* und *Yersinia* pseudotuberculosis spezifischen Sonde, die mit einem konservierten Bereich aus den bakteriellen 16S rDNA-Genen oder einem Teil desselben hybridisiert, und mit den Amplifikationsfragmenten unter Bildung von Hybridnukleinsäuren spezifisch hybridisiert und einer Sonde, die mit einem konservierten Bereich der Plasmide pPla, pFra oder pYV aus *Yersinia pestis* oder eines Teils derselben hybridisiert;
(d) Nachweis der Hybridnukteinsäuren.

Bevorzugte Ausführungsformen sind in Ansprüchen 2-13 dargestellt. Das erfindungsgemäße Verfahren ist sicherer und schneller als bisheriga mikrobiotogische Nachweisverfahren durchführbar und erlaubt den Nachweis von in einer Probe vorhandenen Mikroorganismen der Spezies *Yersinia pestisl Y. pseudotuberculosis* und die Differenzierung der von der Spezies umfaßten Pathovare innerhalb weniger Stunden. Es beinhaltet bevorzugt eine Spezifitätskontrolle, die eine sichere Identifizierung der Reaktionsprodukte erlaubt und damit die Gefahr von falsch positiven bzw. falsch negativen Resultaten minimiert. Das erfindungsgemäße Verfahren ermöglicht eine Automatisierbarkeit der Detektionsreaktion und eine Quantifizierung von *Yersinia pestis*/*Yersinia pseudotuberculosis* im Untersuchungsmaterial Innerhalb nur eines Arbeitstages.

In der nachfolgenden Beschreibung soll unter "Identität" der Grad der Verwandtschaft zwischen zwei oder mehr Nukleinsäuren, Peptiden oder Polypeptiden verstanden werden, der durch die Übereinstimmung zwischen den Sequenzen mittels bekannter Verfahren, z.B. der computergestützten Sequenzvergleiche (Basic local alignment search tool, S.F. Altschul et al., J. Mol. Biol. 215 (1990), 403-410) bestimmt werden kann. Der Prozentsatz der "Identität" ergibt sich aus dem Prozentsatz identischer Bereiche in zwei oder mehr Sequenzen unter Berückslchtigung von Lücken oder anderen Sequenzbesonderheiten. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht beschränkt auf das GAG Programmpaket, einschließlich GAP (Devereux, J., *et al., Nucleic Acids Research* 12 (12): 287 (1984); Genetics Computer Group University of Wisconsin, Madison, (WI)); BLASTP, BLASTN, und FASTA (Altschul, S., et al., J. Mol. Biol. 215:403-410) (1999)). Das BLASTX Programm kann vom National Centre for Biotechnology Information (NCBI) und aus weiteren Quellen bezogen werden (BLAST Handbuch, Altschul S., et al., NCB NLM NIH Bethseda MD 20894; Altschul, S., et al., Mol. Biol. 215:403-410 (1990). Auch der bekannte Smith Waterman-Algorithmus kann zur Bestimmung der Identität herangezogen werden.

Bevorzugte Parameter für den Aminosäuresequenz-Vergleich umfassen die nachstehenden:

| | |
|---|---|
| Algorithmus: | Needleman und Wunsch, J. Mol. Biol. 48:443-453 (1970); |
| Vergleichsmatrix: | BLOSUM 62 aus Henikoff und Henikoff, PNAS USA 89 (1992),10915-10919; |
| Lückenwert: (GAP Penalty) | 12; |
| Lückenlängen-Wert: (GAP Length Penalty) | 4; |
| Homologie-Schwellenwert: (Threshold of Similarity) | 0; |

Das GAP-Programm ist auch zur Verwendung mit den vorstehenden Parametern geeignet. Die vorstehenden Parameter sind die Fehler-Parameter (default parameters) für Aminosäuresequenzvergleichei wobei Lücken an den Enden den Identitätswert nicht verringern. Bei sehr kurzen Sequenzen im Vergleich zur Referenzsequenz kann es weiterhin notwendig sein, den Erwartungswert auf bis zu 100.000 (expectation value) zu erhöhen und gegebenenfalls die Wortlänge (word size) auf bis zu 2 zu verkleinem.

Weitere bespielhafte Algorithmen, Lücken-Öffnungs-Werte (gap opening penalties), Lückenausdehnungs-Werte (gap extension penalties), Vergleichsmatritzen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermitteltete Übereinstimmung von 70% wird im Rahmen dieser Anmeldung als 70% Identität bezeichnet. Entsprechendes gilt für höhere Identitätsgrade.

Unter einer "Hybridisierung" soll die Doppelstrangbildung zweier identischer oder ähnlicher Nukleinsäurefragmente (DNA, RNA, PNA) verstanden werden. Von spezifischer Hybridisierung spricht man, wenn die Hybridisierung unter stringenten Bedingungen durchgeführt wird und zu einer stabilen Hybridnukleinsäure führt. Im Sinne dieser Erfindung weist das Merkmal "Sequenz, die mit einer Sequenz nach (i) spezifisch hybridisiert" auf eine Sequenz hin, die unter stringenten Bedingungen mit der Sequenz nach (i) hybridisiert. Beispielsweise können die Hybridisierungen bei 50 bzw. 55°C mit einer Hybridisierungslösung bestehend aus 2,5 x SSC, 2 x Denhardts Lösung, 10 mM Tris, 1 mM EDTA, pH 7,5 durchgeführt werden. Als Waschbedingungen eignen sich z.B. viermal wiederholte einminütige Waschungen in 0,1 x SSC bis 1,0 x SSC, 2 x Denhardts, 10 mM Tris, 1 mM EDTA, pH 7,6 bei 20 - 55°C.

Unter "mikrobiellem Genom" wird die Gesamtheit der Erbinformation eines Mikroorganismus verstanden. Es erfaßt also das mikrobielle Chromosom sowie die vorhandene episomale DNA.

Eine "Nukleinsäure" ist eine DNA, RNA oder PNA, die entweder durch Isolation aus genomischer DNA oder aus cDNA nach bekannten Standardverfahren (Sambrook *et al.,* 1989) erhalten und aufgereinigt oder künstlich durch bekannte Verfahren (Sambrook *et al.*, 1989), wie z.B. der Oligonukleotid-Synthese generiert wird oder als ribosomale RNA oder mRNA aus dem Organismus isoliert oder als PNA synthetisiert wird. Eine "PNA" ist dabei eine Peptidnukleinsäure, bei der anstelle des Phosphorsäurerückgrates der DNA 2-Aminoethylglycin-Bindungen auftreten. Erfindungsgemäß können in den Nukleinsäuren bis zu 20% der Nukleotide in 10 aufeinanderfolgenden Nukleotiden, bevorzugt jedoch 1 Nukleotid aus einem Block von 10 aufeinanderfolgenden Nukleotiden durch Nukleotide (z.B. Inosin, etc.) ersetzt sein, die in Bakterien nicht natürlich vorkommen.

Die Nukleinsäuren können weiterhin Modifikationen enthalten, die die Erzeugung eines direkt oder indirekt nachweisbaren Signales erlauben. Dem Fachmann sind dabei folgende Modifikationen bekannt:
(i) radioaktive Modifikationen, i.e. radioaktive Phosphorylierung oder radioaktive Markierung mit Schwefel, Wasserstoff, Kohlenstoff, Stickstoff,
(ii) farbige Gruppen (z.B. Digoxygonin, etc.),
(iii) fluoreszierende Gruppen (z.B. Fluorescein, etc.),
(iv) chemolumineszierende Gruppen,
(v) Gruppen zur Immobilisierung an einer festen Phase (z.B. Biotin, Streptag, Antikörper, Antigene, etc.) und/oder
(vi) Gruppen, die eine indirekte oder direkte Reaktion, mit Hilfe von Antikörpern, Antigenen, Enzymen und/oder Substanzen mit Affinität zu Enzymen oder Enzymkomplexen erlauben,
oder Kombinationen von Modifikationen gemäß zwei oder mehr der unter (i) bis (vi) genannten Modifikationen. Als "Modifikation" werden im Sinne dieser Erfindung direkt oder indirekt nachweisbare Gruppen oder Gruppen zur Immobilisierung an eine feste Phase bezeichnet, die an die Nukleinsäure angehängt sind. Direkt nachweisbar sind Metallatome, radioaktive, farbige oder fluoreszierende Gruppen. Indirekt nachweisbar sind immunologisch oder enzymatisch nachweisbare Gruppen, wie z.B. Antigene und Antikörper, Haptene oder Enzyme bzw. enzymatisch wirkende Teile von Enzymen. Diese indirekten Gruppen werden in nachfolgenden Reaktionen nachgewiesen. Bevorzugt sind Haptene, die an ein Oligonukleotid gekoppelt sind und die man in einer anschließenden Antikörperreaktion nachweist.

Als "Primer" werden im Sinne dieser Erfindung Nukleinsäuren verstanden, die z.B. bei einer Polymerase-Kettenreaktion (PCR) spezifisch oder unspezifisch an eine Zielsequenz hybridisieren können. In einer besonderen Ausführungsform im Sinne dieser Erfindung ist ein Primer so modifiziert bzw. markiert, wie oben für Nukleinsäuren beschrieben. Primer umfassen mindestens 10 Nukleotide, bevorzugt 15-50 Nukleotide, besonders bevorzugt 16-26 Nukleotide.

Als "Sonde" wird im Sinne dieser Erfindung eine Nukleinsäure verstanden, die spezifisch mit einer gewünschten Nukleinsäure hybridisiert. Je nach gewünschter Aussage werden Sonden eingesetzt, die entweder mit den Amplifikaten sämtlicher nachzuweisender *Yersinia pestislYersinia pseudotuberculosis* Pathovare, einem einzelnen Pathovar oder der Amplfikationskontrolle reagieren. Eine Sonde ist daher entweder komplementär zu einer Teilsequenz des mikrobiellen Genoms der Spezies *Yersinia pestislYersinia pseudotuberculosis,* die in beiden Pathovaren konserviert ist, oder zu einer in nur einem Pathovar vorkommenden Sequenz, oder zu einer Amplifikationskontrollsequenz. In einer besonderen Ausführungsform im Sinne dieser Erfindung ist eine Sonde modifiziert bzw. markiert, wie oben für Nukleinsäuren beschrieben. Eine Sonde ist mindestens 10 Nukleotide, bevorzugt 15-50 Nukleotide, besonders bevorzugt 20-25 Nukleotide lang.

Als "konserviert' werden Sequenzen bezeichnet, die zu mindestens 70% identisch sind. Ein konservierter Bereich ist dabei ein Bereich, bei dem zwischen den Pathovaren *Yersinia pestis und Yersinia pseudotuberculosis* 70% Identität oder mehr, bevorzugt 80% Identität oder mehr, noch bevorzugter 90% Identität oder mehr, besonders bevorzugt 95% Identität oder mehr und am stärksten bevorzugt 99% Identität oder mehr auftritt.

Erfindungsgemäß wird eine zu untersuchende Probe mit einer Kombination aus mindestens zwei ersten Nukleinsäuren (Primern) in Kontakt gebracht. Bei der Probe kann es sich um Blut, Serum, Plasma, Lymphe, Flüssigkeit aus "Pestbeulen", Speichel, Sputum, Kot, Eiter oder Isolate aus Ektoparasiten handeln.

Bei den mindestens zwei ersten Nukleinsäuren handelt es sich in der Regel um einen Vorwärts- und einen Rückwärtsprimer, die eine Amplifikation des von ihnen eingegrenzten Bereiches erlauben. Sie hybridisieren hierbei mit einem Bereich einer mikrobiellen Nukleinsäure aus der Probe, der in den humanpathogenen Pathovaren der Spezies *Yersinia pestislYersinia pseudotuberculosis* konserviert ist, und somit unabhängig vom vorliegenden Pathovar der Spezies *Yersinia pestislYersinia pseudotuberculosis* eine Amplifikation erlaubt. Die zum Primer komplementären Sequenzen der Pathovare weisen dabei jeweils mindestens 70% Identität mit dem Primer auf. In einer weiteren bevorzugten Ausführungsform der vorliegenden Erfindung werden Primersequenzen verwendet, die mit genomischen Sequenzen aus den Pathovaren *Yersinia pestis*/*Yersinia pseudotuberculosis* hybridisieren, wobei die dem Primer entsprechenden Sequenzen der Pathovare jeweils zu mindestens 80%, bevorzugt 90%, noch mehr bevorzugt 95% und besonders bevorzugt 99% Identität mit dem Primer aufweisen.

Erfindungsgemäß wird in einem weiteren Schritt die zu untersuchenden Probe aus dem ersten Schritt mit einer Kombination aus mindestens zwei ersten Nukleinsäuren (Primern) amplifiziert. Bei diesem Schritt wird mindestens ein Amplifikationsfragment erzeugt. Die Amplifikation kann erfindungsgemäß mit jedem gewünschten Verfahren durchgeführt werden, z.B. einer PCR (US 4 683 195, US 4 683 202, US 4 965 188), einer Ligasekettenreaktion, einer "self-sustained sequence replication" (EP 329 822), einer "transcription based amplification" (EP 310 229), einer "β-RNA-Replicase-System" (US 4 957 858) oder einer isothemen Nukleinsäureamplifikation.

Erfindungsgemäß werden die im vorausgegangenen Verfahrensschritt erhaltenen Amplifikationsfragmente mit mindestens einer zweiten Nukleinsäure (Sonde) In Kontakt gebracht Die Sonde/Sonden hybridisieren hierbei unter Bildung einer Hybridnukleinsäure spezifisch mit mindestens einem Amplifikationsfragment einer mikrobiellen Nukleinsäure, das eine Sequenz der mikrobiellen Nukleinsäure umfaßt, die in den humanpathogenen Pathovaren *Yersinia pestis* und/oder *Yersinia pseudotuberculosis* vorkommt ist Die Hybridisierung erfolgt durch Paarung der Sonden mit Bereichen der mikrobiellen Nukleinsäure, die eine zumindest teilweise komplementäre Basensequenz aufweisen. Durch Auswahl einer geeigneten Sondensequenz kann dann bestimmt werden, ob die Sonde beide Pathovaren der Spezies *Yersinia pestis*/*Yersinia pseudotuberculosis* oder nur eines dieser Pathovare erkennen soll. Auf diese Weise kann entweder der Nachweis humanpathogener Yersinien überhaupt oder aber die Bestimmung eines einzelnen Pathovars erfolgen.

Erfindungsgemäß wird in einem weiteren Schritt die mindestens eine im vorausgegangenen Schritt entstandene Hybridnukleinsäure nachgewiesen, die aus einem Amplifikationsfragment und einer zweiten Nukleinsäure besteht Der Nachweis der Hybridnukleinsäuren kann über verschiedene DNA-Nachweisverfahren erfolgen, wie z.B. Blot-Techniken, Nachweis von radioaktiven Isotopen. Fluoreszenzdetektionsverfahren, optischen Detektionsverfahren oder anderen Detektionsverfahren.

Die erfindungsgen Schnitte des Verfahrens des vol. Erfindung finden sich in Anspruch 1. In einer Ausführungsform der vorliegenden Erfindung wird der Primer aus einem konservierten Bereich des Genoms ausgewählt, der die bakteriellen 16S rDNA Gene enthält. Diese Ausführungsform verwendet auch einen konservierten Bereich aus einem der in *Yersinia pestis* enthaltenen Plasmide pPla (bzw. pPCP1 oder pPst), pFra (bzw. pMT1) und/oder pYV (bzw. pCD1 oder pCad). Hierbei sind Sequenzen aus dem Plasminogen-Aktivator-Gen (pPIa) oder dem F1-Antigen (pFra) bevorzugt Besonders bevorzugt ist der Bereich zwischen Pos. 7359 und 7838 (pPla) bzw. 4879 und 5133 (pFra). Die erfindungsgemäßen Sequenzen nach SEQ ID NO: 1 - 9 sind aus dem Plasminogen-Aktivator-Gen, dem F1-Gen und dem 16S rDNA-Gen abgeleitet und entsprechen folgenden Positionen (vgl. Tab. No. 1):

**Tabelle 1: Ursprung der Sequenzen der SEQ ID NO: 1-9**

| **SEQ ID NO** | **Gen** | **Accession-No** | **Genregion** | **Länge** |
|---|---|---|---|---|
| **1** | Plasminogen-Aktivator Gen | AF053945 | 7859-7378 | 20 |
| **2** | Plasminogen-Aktivator Gen | AF053945 | 7819-7838 | 20 |
| **3** | (F1)-capsulares Antigen, caf1 | X61996 | 5110-5133 | 24 |
| **4** | (F1)-capsulares Antigen, caf1 | X61996 | 4879-4900 | 22 |
| **5** | 16S rDNA | L37604 | 989-1004 | 16 |
| **6** | 16S rDNA | L37604 | 985-1004 | 20 |
| **7** | 16S rDNA | L37604 | 1375-1400 | 26 |
| **8** | 16S rDNA | L37604 | 1375-1391 | 17 |
| **9** | 16S rDNA | L37604 | 1419-1398 | 22 |

SEQ ID NO: 1-9 entsprechen dabei den folgenden Sequenzen (vgl. Tab. No. 2):

**Tabelle 2: Sequenzen der SEQ ID NO: 1-9**

| **SEQ ID NO** | **Sequenz** |
|---|---|
| **1** | ATCTTACTTTCCGTGAGAAG |
| **2** | CTTGGATGTTGAGCTTCCTA |
| **3** | GGATTATTGGTTAGATACGGTTAC |
| **4** | GGTGATCCCATGTACTTAACAT |
| **5** | GGCAGAGATGCTAAAG |
| **6** | ATTTGGCAGAGATGCTAAAG |
| **7** | CTCCCATGGTGTGACGGGCGGTGTGT |
| **8** | TGTGACGGGCGGTGTGT |
| **9** | CTACTTCTTTTGCAACCCACTC |

In den Sequenzen nach SEQ ID NO: 1-13 werden Nukleotide folgendermaßen abgekürzt: G = Guanosin, A = Adenosin, T = Thymidin, C = Cytosin.

Erfindungsgemäß wird eine Kombination aus mindestens zwei ersten Nukleinsäuren (Primspaaren) eingesetzt Die eingesetzte Kombination aus mindestens zwei ersten Nukleinsäuren wird dabei so gewählt, dass sie als Primer in einer Amplifikationsreaktion einsetzbar sind, d.h. eine Nukleinsäure hybridisiert an einen ersten konservierten Bereich des ersten Stranges der Ziel-DNA und die andere Nukleinsäure an einen zweiten konservierten Bereich des zum ersten Strang komplementären DNA-Stranges, wobei der gewünschte Zielbereich der DNA eingeschlossen wird. Beide Nukleinsäuren weisen jeweils eine Länge von 15 - 25 Nukleotiden auf. In einer weiteren bevorzugten Ausführungsform der Erfindung wird eine Kombination aus mindestens zwei Paaren von ersten Nukleinsäuren gemäß dieser Erfindung eingesetzt, die zusammen also zu zwei amplifizierten Fragmenten führen. In einer besonders bevorzugten Ausführungsform hybridisiert ein erstes Paar von Nukleinsäuren mit der bakteriellen 16S rDNA, während ein zweites und gegebenenfalls ein drittes, oder weiteres Primerpaar mit Sequenzen aus pPla, pFra oder pYV hybridisiert.

Jede erste Nukleinsäure (Primer) Ist ausgewählt aus: (i) einer Nukleinsäure, die eine Nukleinsäuresequenz nach SEQ ID NO: 1-9 umfaßt oder ein 15 bis 25 Nukleotide langes Fragment derselben; (II) einer Nukleinsäure, die mit einer Nukleinsäure nach (I) spezifisch hybridisiert; (III) einer Nukleinsäure, die mindestens 70% Identisch mit einer Nukleinsäure nach (i) oder (II) Ist oder (iv) einer Nukleinsäure, die komplementär zu einer Nukleinsäure nach (i) bis (iii) ist

In einer bevorzugten Ausführungsform der Erfindung umfaßt das Verfahren zur Amplifikation eine Polymerase-Kettenreaktion (PCR). Die Pölymerase-Kettenreaktion kann auch als eine Multlplex-PCR durchgeführt werden. Die "Multiplex-PCR" ist ein Nachweis verschiedener Zielmoleküle in einer einzigen Polymerase-Kettenreaktion, wobei ein Gemisch von Primern verwendet wird. In einer bevorzugten Ausführungsform der Erfindung werden in einer Multiplex-PCR vier erste Nukleinsäuren als Primer eingesetzt Auch eine online- oder realtime-PCR sind möglich. Bei der online- oder realtime-PCR wird das Resultat der Amplifikation simultan mit seiner Entstehung aufgezeichnet. Im Idealfall, wie z.B. beim LightCycler, kann die Amplifikation auch bereits in Echtzeit detektiert werden. Ein wesentlicher Vorteil der realtime-PCR liegt somit in der Schnelligkeit, mit der das Resultat erhalten wird. Dadurch, dass sekundäre Arbeitsschritte, wie die Detektion der Amplifikationsprodukte mittels ELISA oder Gelelektrophorese entfallen, wird der Arbeitsaufinrand beträchtlich reduziert Schließlich bietet die realtime-PCR den Vorteil, dass sie eine "echte" Quantifizierung der in der Probe vorhandenen DNA und somit der Anzahl der Mikroorganismen erlaubt Wenn anstelle von DNA RNA als Zielmolekül in einer RT-PCR detektiert wird, kann zwischen lebenden und toten *Yersinia* pestis-Keimen differenziert werden. Auch die online-PCR kann mit einem oder mehreren der Primer (SEQ ID NO: 1-9) durchgeführt werden. Im weiteren ist für die Funktionalität der önline-PCR das Vorhandensein einer oder mehrerer Sonden, z.B. der SEQ ID NO: 10-13, im PCR-Reaktionsgemisch entscheidend. Die online-PCR selbst kann auf verschiedene Weisen durchgeführt werden. Im Wesentlichen unterscheiden sie sich von anderen PCR-Verfahren nur durch das Zustandekommen des Fluoreszenzsignals. Verwendet werden können z.B. der 5'-Nuklease-Assay mit TaqMan-Sonden oder Molecular Beacons. Eine weitere Möglichkeit besteht darin, LightCycler-Sonden zu verwenden. In letzterem Fall werden zwei fluoreszenzmarkierte Sonden eingesetzt und das Signal entsteht durch einen Energietransfer von dem Farbstoff der einen Sonde zu dem Farbstoff einer benachbarten Sonde. Aus diesem Grunde wird hier neben den Sonden der SEQ ID: 10 - 13 eine "Nachbarsonde" von den bekannten PCR-Zielsequenzen von *Yersinia pestis* ausgewählt werden.

in einer bevorzugten Ausführungsform der vorliegenden Erfindung werden zum Nachweis humanpathogener Yersinien Sondensequenzen verwendet, die mit genomischen Sequenzen aus den Pathovaren *Yersinia pestis* und *Yersinia pseudotuberculosis* hybridisieren, wobei die zur Sonde komplementären Sequenzen der Pathovare untereinander zu mindestens 80% identisch sind. Bevorzugt werden dabei Sondensequenzen verwendet, die mit genomischen Sequenzen aus den Pathovaren *Yersinia pestis* und *Yersinia pseudotuberculosis* hybridisieren, wobei die zur Sonde komplementären Sequenzen der Pathovare untereinander zu mindestens 90%, bevorzugt 95%, besonders bevorzugt 99% identisch sind. In einer bevorzugten Ausführungsform der vorliegenden Erfindung wird die Sonde aus einem konservierten Bereich des Genoms ausgewählt, der die bakteriellen 16S rDNA Gene enthält. Eine weitere bevorzugte Ausführungsform verwendet einen konservierten Bereich aus einem der in *Yersinia pestis* enthaltenen Plasmide pPla (bzw. pPCP1 oder pPst), pFra (bzw. pMT1) und/oder pYV (bzw. pCD1 oder pCad). Hierbei sind Sequenzen aus dem Plasminogen-Aktivator-Gen (pPla) oder dem F1-Antigen (pFra) bevorzugt.

Für den Nachweis eines einzelnen Pathovars sind dagegen Sonden bevorzugt, die eine Identität von 80% oder mehr, bevorzugt 90%, 95%, 99% oder mehr mit nur einem der Pathovare aufweisen, während die Identität mit dem anderen Pathovar entsprechend geringer sein muß, um eine selektive Hybridisierung beobachten zu können. Bevorzugt beträgt der Unterschied im Identitätsgrad mindestens 5%, besser aber mindestens 10% oder 15%, und besonders bevorzugt mehr als 20 oder 25%.

Für Sonden besonders bevorzugt ist der Bereich zwischen Pos. 7583 und 7602 (pPla) bzw. 5008 und 5027 (pFra). Die erfindungsgemäß bevorzugten Sequenzen nach SEQ ID NO: 10 bis 12 sind aus dem Plasminogen-Akfivator-Gen, dem F1-Gen und dem 16S rDNA-Gen abgeleitet und entsprechen folgenden Positionen (vgl. Tab. 3):

**Tabelle 3: Ursprung der Sequenzen der SEQ ID NO: 10-12**

| **SEQ ID NO** | **Gen** | **Accession-No.** | **Genregion** | **Länge** |
|---|---|---|---|---|
| **10** | 16S rDNA | L37604 | 1178-1198 | 21 |
| **11** | (F1)-capsulares Antigen, caf1 | X61996 | 5008-5027 | 20 |
| **12** | Plasminogen-Aktivator Gen | AF053945 | 7583-7602 | 20 |

SEQ ID NO:10-12 entsprechen dabei den folgenden Sequenzen (vgl. Tab. 4):

**Tabelle 4: Sequenzen der SEQ ID NO: 10-12**

| **SEQ ID NO** | **Sequenz** |
|---|---|
| **10** | AGTCATCATGGCCCTTACGAG |
| **11** | GATGACGTCGTCTTGGCTAC |
| **12** | GGTCTGCAATATCGCTTCTG |

In den Sequenzen nach SEQ ID NO: 1 -12 werden Nukleotide folgendermaßen abgekürzt G = Guanosin, A = Adenosin, T = Thymidin, C = Cytosin.

Sonden weisen eine Länge von 15 bis 20 Nukleotiden auf. Bevorzugte Sonden sind ausgewählt aus:
(v) einer Nukleinsäure, die eine Nukleinsäuresequenz nach SEQ ID NO: 10-12 umfaßt oder ein 15 bis 20 Nukleotide langes Fragment derselben;
(vi) einer Nukleinsäure, die mit einer Nukleinsäure nach (v) spezifisch hybridisiert;
(vii) einer Nukleinsäure, die mindestens 80% identisch mit einer Nukleinsäure nach (v) oder (vi) ist; oder
(viii) einer Nukleinsäure, die komplementär zu einer Nukleinsäure nach (v) bis (vii) ist.

Sonden können weiterhin Modifikationen enthalten, die die Erzeugung eines direkt oder indirekt nachweisbaren Signales erlauben.

Die Sonden der SEQ ID NO: 10-12 besitzen für die Pathovare *Yersinia pestis* und *Yersinia pseudotuberculosis* eine unterschiedliche Spezifität (vgl. Tab. 6). So kann mit den Sonden der SEQ ID NO: 11-12 das Pathovar *Yersinia pestis* spezifisch nachgewiesen werden, während hingegen mit der Sonde der SEQ ID NO: 10 die Pathovare *Yersinia pestis und Yersinia pseudotuberculosis* nachgewiesen werden können. Dadurch ist eine Differenzierung zwischen den Pathovaren *Yersinia pestis* und *Yersinia pseudotuberculosis* möglich.

Um falsch negative Ergebnisse zu vermeiden, wird in einer bevorzugten Ausführungsform der Erfindung bei der Amplifikation eine Amplifikationskontroll-Nukleinsäure zugesetzt Dementsprechend wird nicht nur die mikrobielle Nukleinsäure oder ein Teil derselben amplifiziert sondern auch die Amplifikadonskontroll-Nukleinsäure, wodurch mindestens je ein Amplifikationsfragment erzeugt wird. Die Amplifikationskontroll-Nukleinsäure, z.B, ein DNA-Fragment, ist hierbei ein "internes Standard Molekül", das als Indikator für die Effektivität der Reaktion dient (BallagiPordany, Belak, 1996, *Mol. Cell. Probes* 10, .159-164). Es wird In einer definierten Menge der Amplifikationsreaktion zugesetzt und parallel amplifiziert. Die Amplifikationskontroll-Nukleinsäure ist bevorzugt einzel- oder doppelsträngig und kann unbegrenzt lang sein. Bewährt haben sich Amplifikationskontroll-Nukleinsäuren mit einer Länge von bis zu tausend Nukleotiden. In einer bevorzugten Ausführungsform dieser Erfindung enthält die Amplifikationskontroll-Nukleinsäure eine zu der Sequenz der SEQ ID NO: 13 oder eines Teils derselben identische oder komplementäre Sequenz

In einer weiteren bevorzugten Ausführungsform dieser Erfindung findet die Amplifikation als kompetitive PCR statt. Bei der kompetitiven PCR werden die Ziel-DNA und die Amplifikationskontroll-DNA mit demselben Primerpaar amplifiziert. Bei einer besonders bevorzugten Ausführungsform der Erfindung sind der 3'- und 5'-Bereich der Amplifikationskontroll-DNA so ausgestaltet, dass diese mit einem für die Amplifikation der Ziel-DNA ausgesuchten Primerpaar amplifiziert werden kann. In diesem Fall spricht man von einer kompetitiven Amplifikationskontrolle, da die Ziel-DNA und die Kontroll-DNA mit dem gleichen Primerpaar amplifiziert werden. In einer bevorzugten Ausführungsform dieser Erfindung wird die Amplfikationskontroll-Nukleinsäure mit Hilfe von mindestens zwei der Primer der SEQ ID NO: 5-9 amplifiziert.

In einer besonderen Ausführungsform des erfindungsgemäßen Verfahrens wird bei Verwendung einer Amplifikationskontrolle die amplifizierte Amplifikationskontroll-Nukleinsäure mit einer Sonde In Kontakt gebracht, die hierbei spezifisch mit mindestens einem Amplifikationsfragment der Amplifikationskontroll-Nukleinsäure hybridisiert Die Sonde wird ausgewählt aus:
(ix) einer Nukleinsäure, die eine Nukleinsäuresequenz nach SEQ ID NO: 13 umfaßt; oder ein 15 bis 19 Nukleotide langes Fragment derselben;
(x) einer Nukleinsäure, die mit einer Nukleinsäure nach (ix) spezifisch hybridisiert:
(xi) einer Nukleinsäure, die mindestens 80% identisch mit einer Nukleinsäure nach (ix) oder (x) ist; oder
(xii) einer Nukleinsäure, die komplementär zu einer Nukleinsäure nach (ix) bis (xi) ist.

Eine Sonde nach SEQ ID NO: 13 kann weiterhin Modifikationen enthalten, die die Erzeugung eines direkt oder indirekt nachweisbaren Signales erlauben. Die Sonde nach SEQ ID NO: 13 kann getrennt oder in Kombination mit einer oder mehreren Sonden nach SEQ ID NO: 10-12 verwendet werden. SEQ ID NO: 13 entspricht dabei folgender Sequenz:

SEQ ID NO: 13 GACTACGGAATTCCGCTGTC

Erfindungsgemäß erfolgt der Nachweis von mindestens einer Hybridnukleinsäure, die aus einem Amplifikationsfragment und einem im vorangegangenen Schritt eingebrachten zweiten Nukleinsäure besteht. Der Nachweis der Hybridnukleinsäuren kann über verschiedene DNA-Nachweisverfahren wie z.B. Blot-Techniken, Fluoreszenzdetektionsverfahren, optischen Detektionsverfahren oder anderen Detektionsverfahren erfolgen. In einer bevorzugten Ausführungsform erfolgt die Detektion der Hybridnukleinsäuren über ELISA-Techniken. In einer weiteren bevorzugten Ausführungsform erfolgt die Detektion der Hybridnukleinsäure über Southem Blot-Verfahren.

Eine Vervielfältigung der mikrobiellen Nukleinsäure oder eines Teils derselben und eine anschließende Detektion dieser Moleküle kann z.B. mittels Hybridisierung mit markierten spezifischen Sonden erfolgen. In einer Multiplex-PCR können Nukleinsäuren eingesetzt werden, die es ermöglichen, von mehreren oder gar allen der relevanten Stämme, Subspezies oder Spezies sowie verschiedenen Zielmolekülen ein Amplifikationsprodukt zu erhalten. Die Spezifität des Nachweises wird durch die anschließende Hybridisierungsreaktion mit spezifischen Sonden erreicht. Auf diese Art und Weise können *Yersinia pestis* und *Y. pseudotuberculosis* in Gegenwart einer Amplifikationskontrolle simultan in einer einfachen Kombination aus Amplifikations- und Detektionsreaktion erfaßt werden. Diese Art der Amplifikation und Detektion ermöglicht die Automatisierbarkeit der Detektionsreaktion, so dass ein hoher Probendurchsatz ermöglicht wird. Es kann beispielsweise ein PCR-ELISA-Nachweisverfahren eingesetzt werden, bei dem die entsprechenden Sonden in verschiedene Kavitäten einer Mikrotiterplatte gebunden werden, in denen anschließend die Hybridisierung und der Nachweis der markierten Amplifikate geschieht. Der Nachweis kann auch durch die Verwendung eines Mikroarrays erfolgen, auf dem mehrere Sonden immobilisiert sind, wodurch die Nachweisreaktion schnell und ohne großen Aufwand durchgeführt werden kann. Die erfindungsgemäßen Nukleinsäuren können somit zum Nachweis und/oder zum Identifizieren und/oder Charakterisieren von *Yersinia pestis* bzw. *Y. pseudoluberculosls* verwendet werden. Eine weitere Verwendung können die hier beschriebenen Primer und/oder Sonden auch bei der Detektion der beschriebenen Keime In verschiedenen Proben wie z.B. in Umwelt- oder klinischen Proben etc. finden.

Die Erfindung umfaßt weiterhin einen Kit für den Nachweis von Mikroorganismen der Spezies *Yersinia pestis*/*Yersinia pseudotuberculosis* sowie für die Differenzierung zwischen *Yersinia pestis* und *Yersinla pseudotuberculosis* wie in Ausprüchen 14-19 definiert. Die Erfindung umfaßt weiterhin in einer besonderen Form der Ausführung einen Kit für den Nachweis von Mikroorganismen der Spezies *Yersinia pestis*/*Yersinia pseudotuberculosis* sowie für die Differenzierung zwischen *Yersinia pestis* und *Yersinia pseudotuberculosis,* enthaltend zwei oder mehr Nukleinsäuren und eine Amplifikationskontrolle für die Amplifikationsreaktion, wie in Ansprüchen 20 -21 definiert.

Die Figuren und Beispiele erläutern die Erfindung:

### Figurenbeschreibung

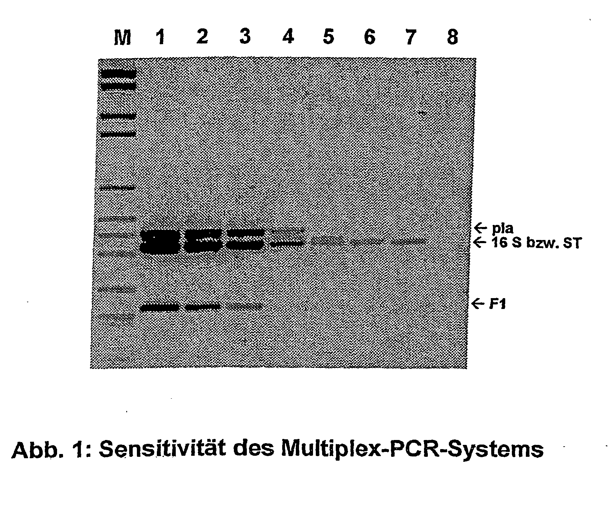

- Figur 1:: Sensitivität des Multiplex-PCR-Systems
Fig. 1 zeigt eine invertierte Darstellung der Amplifikate von genomischer DNA (*Y. pestis)* nach der Amplifikation und gelelektrophoretischer Auftrennung;
Legende (Spuren von links nach rechts):
M: Marker, Spur 1 bis 7: *Y. pestis,* 1 ng bis 1 fg initial eingesetzte genomische DNA, Spur 8: Negativkontrolle;
pla: Amplifikat Plasminogen Aktivator Gen;
16S bzw. ST: Amplifikat 16 S bzw. Kontroll-DNA;
F1: Amplifikat F1 Antigen;

### Beispiele

### Beispiel 1 : Nachweis von humanpathogenen Bakterien mit der Polymerase-Kettenreaktion

### I. Amplifikation

Aus abgetöteten Reinkulturen der in Tabelle 5 aufgeführten Bakterien wurde über bekannte Standardverfahren genomische DNA isoliert. Verdünnungen (Konzentration im Bereich von ca. 1 ng -1 pg) dieser Präparationen wurden anschließend in eine Multiplex-PCR mit folgender Zusammensetzung eingesetzt:
Primer 1 = SEQ ID NO: 1
Primer 2 = SEQ ID NO: 2
Primer 3 = SEQ ID NO: 3
Primer 4 = SEQ ID NO: 4
Primer 5 = SEQ ID NO: 6
Primer 6 = SEQ ID NO: 9

| **Komponenten:** | **Volumen [µl]** | **Konzentration im Ansatz** |
|---|---|---|
| H₂O | ad 25 | - |
| PCR-Puffer | 2,5 | 1x-Konz. |
| MgCI₂ | 2,0 | 0,5-5,0 mM |
| dTTP-Mix | 0,5 | je 10-100 µM |
| Primer: | | |
| SEQ ID NO: 1 | | |
| SEQ ID NO: 2* | | |
| SEQ ID NO: 3* | je 0,5 | 0,2 -1,0 µM |
| SEQ ID NO: 4 | | |
| SEQ ID NO: 6 | | |
| SEQ ID NO: 9* | | |
| Taq-Polymerase | 0,2 | 1-5 U/µl |
| DNA-Extrakt/-Eluat der Probe | 1,0 | var. |
| Kontroll-DNA | 1,0 | var. |
| Σ | 25 µl | |

| | | |
|---|---|---|
| * = Primer sind 5'-Digoxigenin markiert | | |

Die PCR wurde unter folgenden Bedingungen in dem Thermocycler Perkin Elmer, Modell 9700 durchgeführt:

| **Schritt** | **Zeit** | **Temperatur** |
|---|---|---|
| initiale Denaturierung | 5 min | 95°C |
| "touch down": 5x (- 2 °C) | | |
| Denaturierung | 20 - 60 sec | 94°C |
| Anlagerung | 20 - 60 sec | 68°C - 58°C |
| Synthese | 30 - 90 sec | 72 °C |
| | 30-35x | |
| Denaturierung | 20 - 60 sec | 94°C |
| Anlagerung | 20 - 60 sec | 58°C |
| Synthese | 30 - 90 sec | 72°C |
| finaler Syntheseschritt | 5 min | 72°C |
| Endtemperatur | | 4°C-10°C |

Primer 3 (SEQ ID NO: 3) und Primer 4 (SEQ ID NO: 4) wurden durch Sequenzvergleich bekannter F1 Antigen Sequenzen (GenBank Sequence Database des NCBI) bestimmt. Sie hybridisieren an hochkonservierte Sequenzabschnitte im F1 Antigen Genbereich. Primer 5 (SEQ ID NO: 6) und Primer 6 (SEQ ID NO: 7 bzw. 9) wurden durch Sequenzvergleich bekannter 16S rDNA Sequenzen (GenBank Sequence Database des NCBI) sowie eigener Sequenzdaten bestimmt.

### II. Detektion im PCR-ELISA

Die Detektion erfolgt im PCR-ELISA. Dazu werden je verwendeter Sonde 5 µl Amplifikat mit 5 µl Denaturierungspuffer (pH 14; 100-200 mM NaOH, 10-25 mM EDTA) versetzt und 10 min. bei Raumtemperatur inkubiert. Je Probe werden 1,5 pmol der jeweiligen biotinylierten Sonde (SEQ ID NO: 10-13) in 100 µl Hybridisierungspuffer (pH 7,5; 2 - 5x SSC, 1- 5x Denhardts Lösung, 5- -10 mM Tris, 0,1 -1,5 mM EDTA) pipettiert. Nach der Denaturierung wird pro Kavität der Mikrotiterplatte 10 µl des Denaturierungsansatzes sowie 100 µl der jeweiligen Sonden-Hybridisierungspuffer-Mischung in die Kavitäten einer mit Streptavidin beschichteten Mikrotiterplatte überführt. Anschließend erfolgt die 30minütige Inkubation bei Hybridisierungstemperatur (45 - 55°C). Ist die Hybridisierung abgeschlossen, wird der Hybridisierungsansatz entfernt und 4x mit 200 µl Waschpuffer 1 (WP1: pH 7,6; 0,1 -1 x SSC, 1 - 5x Denhardts, 5 - 10 mM Tris, 0,1 - 1,5 EDTA) jeweils für 1 bis 4 min. bei Hybridisierungstemperatur gewaschen. Anschließend werden 100 µl einer nach Herstellerangaben verdünnten Lösung eines mit einer Meerrettichperoxidase konjugierten Anti-Digoxigenin-Antikörpers zugegeben (Boehringer Mannheim). Das Konjugat wird in Waschpuffer 2 (WP2: pH 7,5; 50 -100 mM Tris, 100 - 200 mM NaCl, 0,01 - 0,1 % Tween 20, 0,1 -1,0 % Blocking-Reagenz, 50 -120 µg/ml Heringssperma) verdünnt. Anschließend erfolgt die Antikörper-Inkubation bei 37°C für 30 min. Danach wird 4x mit 200 µl Waschpuffer 2 gewaschen (bei Raumtemperatur). Nach dem Waschen werden 100 µl POD-Substrat (Boehringer Mannheim) zugegeben und 15 min. bei RT inkubiert. Anschließend wird die Farbreaktion mit 100 µl 0,3-0,7 M H₂SO₄ abgestoppt und bei 450 nm gegen 650 nm im ELISA-Reader gemessen.

### 111. Auswertung

Gemäß dem oben aufgeführten Detektionsprotokoll wurde der Nachweis für alle untersuchten Bakterien unter Verwendung der entsprechenden spezifischen Sonden geführt. Als spezifische Sonden wurden für *Yersinia pestis* und *Y. pseudotuberculosis* SEQ ID NO: 10 -13 eingesetzt.

War die gemessene Extinktion größer als 0.50, so wurde das Ergebnis positiv bewertet. Die Ergebnisse des PCR-ELISA sind in Tabelle 6 dargestellt.

**Tabelle 5: Verwendete Mikroorganismen**

| **Spezies** | **Stamm-Nr/biovar** | **Kürzel** |
|---|---|---|
| *Yersinia aldovae* | ATCC 35236 | |
| *Yersinia enterocolitica* | DSM 4780 | |
| *Yersinia frederiksenii* | ATCC 33641 | |
| *Yersinia intermedia* | ATCC 29909 | |
| *Yersinia kristensenii* | ATCC 33638 | |
| *Yersinia moliaretii* | ATCC 43969 | |
| *Yersinia pseudotuberculosis* | DSM 8992 | |
| *Yersinia pseudotuberculosis* | NC 1102 | |
| *Yersinia pseudotuberculosis* | NC 824 | |
| *Yersinia pseudotuberculosis* | NC 1779 | |
| *Yersinia pseudotuberculosis* | NC 9507 | |
| *Yersinia pseudotuberculosis* | NC 10277 | |
| *Yersinia pseudotuberculosis* | NC 10278 | |
| *Yersinia pseudotuberculosis* | NC 8580 | |
| *Yersinia pseudotuberculosis* | NC 8579 | |
| *Yersinia pestis* | ATCC 19428 | 172 |
| *Yersinia pestis* | biotype "O" | EV76 |
| *Yersinia pestis* | biotype "O" | 6/69 |
| *Yersinia pestis* | biotype "O" | A1122 |
| *Yersinia pestis* | biotype "O" | M23 |
| *Yersinia pestis* | biotype "O" | G32 |
| *Yersinia pestis* | biotype "A" | Yokuhama |
| *Yersinia pestis* | biotype "A" | Kuma |
| *Yersinia pestis* | biotype "M" | KIM |
| *Yersinia rohdei* | ATCC 43380 | |
| *Yersinia ruckeri* | ATCC 29473 | |

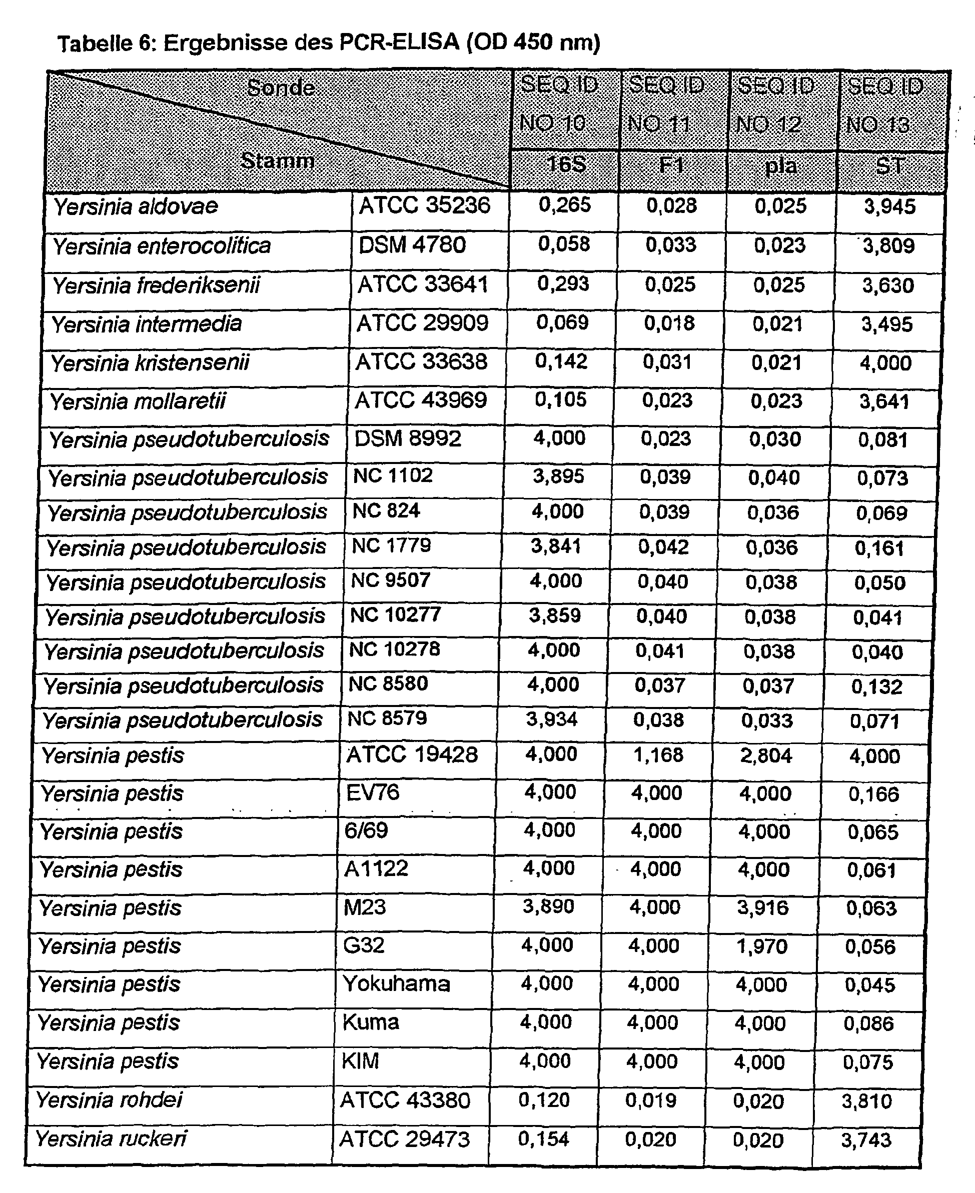

Die in Tabelle 6 dargestellten Ergebnisse zeigen, dass die untersuchten Stämme der Spezies *Yersinia pestis* bzw. *Y. pseudotuberculosis* identifiziert wurden.

### Beispiel 2: Sensitivität des Nachweises von humanpathogenen Bakterien mit der Polymerase-Kettenreaktion

Amplifikation und Detektion im PCR-ELISA und Auswertung wurden entsprechend der Beschreibung in Beispiel 1 durchgeführt. Als Probenmaterial wurde abweichend von Beispiel 1 die aus dem Stamm *Y. pestis* ATCC 19428 isolierte genomische DNA in dekadischen Verdünnungen in eine Multiplex-PCR eingesetzt. Die Ergebnisse sind in Figur 1 und Tabelle 7 dargestellt.

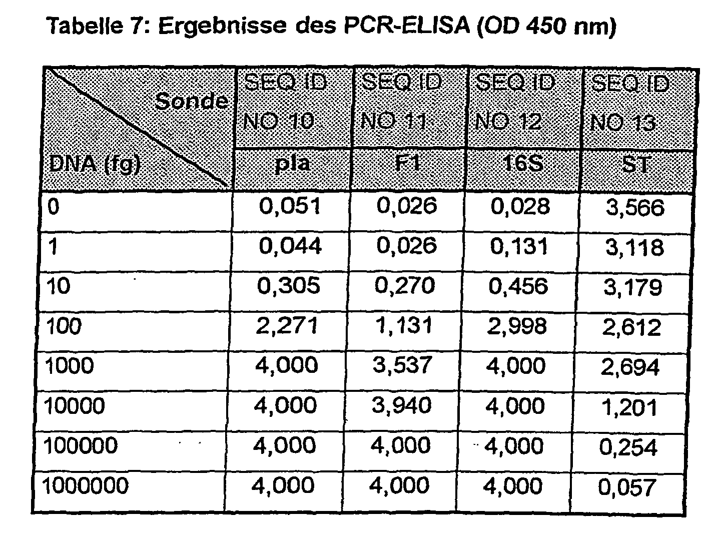

## Patentansprüche

1. Verfahren zum Nachweis von Mikroorganismen der Spezies *Yersinia pestis* und *Yersinia pseudotuberculosis* und zur Differenzierung zwischen *Yersinia pestis* und *Yersinia pseudotuberculosis* in einer Probe, wobei das Verfahren folgende Schritte umfasst:
(a) Inkontaktbringen der Probe mit einer Kombination aus mindestens zwei Primerpaaren, die mit einem in der Spezies *Yersinia pestis*/*Yersinia pseudotuberculosis* konservierten Bereich ihres mikrobiellen Genoms oder eines Teils desselben hybridisieren, wobei ein erstes Primerpaar mit einem konservierten Bereich eines bakteriellen 16S rDNA-Genes oder eines Teils desselben hybridisiert und ein zweites Primerpaar mit einem konservierten Bereich der Plasmide pPla, pFra oder pYV aus *Yersinia pestis* oder eines Teils derselben hybridisiert;
(b) Amplifikation der mikrobiellen Genome oder eines Teils derselben zur Erzeugung von mindestens zwei Amplifikationsfragmenten;
(c) Inkontaktbringen der in Schritt (b) erhaltenen Amplifikationsfragmente mit mindestens einer für *Yersinia pestis* und *Yersinia pseudotuberculosis* spezifischen Sonde, die mit einem konservierten Bereich aus den bakteriellen 16S rDNA-Genen oder einem Teil desselben hybridisiert, und mit den Amplifikationsfragmenten unter Bildung von Hybridnukleinsäuren hybridisiert; und einer Sonde, die mit einem konservierten Bereich der Plasmide pPla, pFra oder pYV aus *Yersinia pestis* oder eines Teils derselben hybridisiert;
(d) Nachweis der Hybridnukleinsäuren.

2. Verfahren nach Anspruch 1, **dadurch gekennzeichnet, dass** die mindestens zwei Primerpaare aus der folgenden Gruppe von Nukleinsäuren ausgewählt sind:
(i) einer Nukleinsäure, die eine Nukleinsäuresequenz nach SEQ ID NO: 1-9 umfasst oder ein 15 bis 25 Nukleotide langes Fragment derselben;
(ii) einer Nukleinsäure, die mit einer Nukleinsäure nach (i) spezifisch hybridisiert;
(iii) einer Nukleinsäure, die mindestens 70% identisch mit einer Nukleinsäure nach (i) oder (ii) ist;
(iv) einer Nukleinsäure, die komplementär zu einer Nukleinsäure nach (i) bis (iii) ist.

3. Verfahren nach Anspruch 1, **dadurch gekennzeichnet, dass** als Sonde eine oder mehrere Nukleinsäuren verwendet werden, die aus der folgenden Gruppe ausgewählt sind:
(i) einer Nukleinsäure, die eine Nukleinsäuresequenz nach SEQ ID NO: 10-12 umfasst oder ein 15 bis 20 Nukleotide langes Fragment derselben;
(ii) einer Nukleinsäure, die mit einer Nukleinsäure nach (i) spezifisch hybridisiert;
(iii) einer Nukleinsäure, die mindestens 80 % identisch mit einer Nukleinsäure nach (i) oder (ii) ist;
(iv) einer Nukleinsäure, die komplementär zu einer Nukleinsäure nach (i) bis (iii) ist.

4. Verfahren nach einem der Ansprüche 1-3. **dadurch gekennzeichnet, dass** die Nukleinsäure eine DNA, RNA oder PNA ist.

5. Verfahren nach einem der Ansprüche 1-4, **dadurch gekennzeichnet, dass** bis zu 20 % der Nukleotide, mindestens jedoch 1 Nukleotid in 10 aufeinander folgenden Nukleotiden durch Nukleotide ersetzt ist, die in Bakterien nicht natürlich vorkommen.

6. Verfahren nach einem der Ansprüche 1 - 5, **dadurch gekennzeichnet, dass** in die Nukleinsäure zur Erzeugung eines direkt oder indirekt nachweisbaren Signals eine Modifikation eingeführt ist, wobei die Modifikation bestehen kann aus (i) einer radioaktiven Markierung, (ii) farbigen Gruppen, (iii) fluoreszierenden Gruppen, (iv) chemolumineszierenden Gruppen, (v) Gruppen zur Immobilisierung an einer festen Phase und/oder (vi) Gruppen, die eine indirekte oder direkte Nachweisreaktion, mit Hilfe von Antikörpern, Antigenen, Enzymen und/oder Substanzen mit Affinität zu Enzymen oder Enzymkomplexen erlauben, oder eine Kombination von Modifikationen gemäß zwei oder mehr der unter (i) bis (vi) genannten Modifikationen.

7. Verfahren nach einem der Ansprüche 1 - 6, **dadurch gekennzeichnet, dass** das Amplifizieren eine Polymerase-Kettenreaktion (PCR) umfasst.

8. Verfahren nach Anspruch 7, **dadurch gekennzeichnet, dass** die Polymerase-Kettenreaktion (PCR) eine Multiplex-PCR umfasst.

9. Verfahren nach Anspruch 7, **dadurch gekennzeichnet, dass** die Polymerase-Kettenreaktion (PCR) eine online- oder realtime-PCR umfasst.

10. Verfahren nach einem der Ansprüche 1 bis 6, **dadurch gekennzeichnet, dass** das Amplifizieren eine Ligase-Kettenreaktion, eine isotherme Nukleinsäure-amplifikation oder eine Qß-Replikation umfasst.

11. Verfahren nach einem der Ansprüche 1 bis 10, **dadurch gekennzeichnet, dass** bei der Amplifikation eine Amplifikationskontrolle durchgeführt wird.

12. Verfahren nach Anspruch 11, **dadurch gekennzeichnet, dass** die Amplifikationskontrolle eine DNA-Sequenz von maximal 1000 Nukleotiden umfasst.

13. Verfahren nach Anspruch 12, **dadurch gekennzeichnet, dass** die DNA-Sequenz der Amplifikationskontrolle aus der folgenden Gruppe ausgewählt ist:
(i) einer Nukleinsäure, die eine Nukleinsäuresequenz nach SEQ ID NO: 13 umfasst oder 15 bis 19 Nukleotide langes Fragment derselben;
(ii) einer Nukleinsäure, die mit einer Nukleinsäure nach (i) spezifisch hybridisiert;
(iii) einer Nukleinsäure, die mindestens 80 % identisch mit einer Nukleinsäure nach (i) oder (ii) ist;
(iv) einer Nukleinsäure, die komplementär zu einer Nukleinsäure nach (i) bis (iii) ist.

14. Ein Kit für den Nachweis von Mikroorganismen der Spezies *Yersinia pestis*/*Yersinia pseudotuberculosis* sowie für die Differenzierung zwischen *Yersinia pestis* und *Yersinia pseudotuberculosis,* enthaltend
a) zwei Primerpaare, wobei
ein erstes Primerpaar mit einem konservierten Bereich eines bakteriellen 16S rDNA-Gens oder eines Teils desselben hybridisiert und ausgewählt ist aus der Gruppe umfassend
(i) Nukleinsäuren, die eine Nukleinsäuresequenz nach SEQ ID NO: 5 bis 9 umfassen oder ein 15 bis 25 Nukleotide langes Fragment derselben;
(ii) Nukleinsäuren, die mit einer Nukleinsäure nach (i) spezifisch hybridisieren;
(iii) Nukleinsäuren, die mindestens 70 % identisch mit der Nukleinsäure nach (i) oder (ii) sind, und
(iv) Nukleinsäuren, die komplementär mit einer Nukleinsäure nach (i) bis (iii) sind,
wobei die eingesetzte Kombination aus den Primern so gewählt wird, dass sie in einer Amplifikationsreaktion einsetzbar sind, d. h. eine Nukleinsäure hybridisiert mit einem ersten konservierten Bereich des ersten Stranges der Ziel-DNA und die andere Nukleinsäure an einen zweiten konservierten Bereich des zum Strang komplementären DNA-Stranges, wobei der gewünschte Zielbereich der DNA eingeschlossen wird; und ein zweites Primerpaar, das mit einem konservierten Bereich der Plasmide pPla, pFra oder pYV aus *Yersinia pestis* oder eines Teils derselben hybridisiert und ausgewählt ist aus
(i) Nukleinsäuren, die eine Nukleinsäuresequenz aus SEQ ID NO: 1 bis 4 umfasst oder ein 15 bis 25 Nukleotide langes Fragment derselben, sowie
(ii) Nukleinsäuren, die mit einer Nukleinsäure nach (i) spezifisch hybridisieren;
(iii) Nukleinsäuren, die mindestens 70 % identisch mit der Nukleinsäure nach (i) oder (ii) sind, sowie
(iv) Nukleinsäuren, die komplementär mit einer Nukleinsäure nach (i) bis (iii) sind; und
b) zwei Sonden, wobei
eine Sonde mit einem konservierten Bereich aus den bakteriellen 16S rDNA-Genen oder eines Teils desselben hybridisiert und aus
(i) Nukleinsäuren ausgewählt ist, die eine Nukleinsäuresequenz aus SEQ ID NO: 10 umfassen, oder ein 15 bis 20 Nukleotide langes Fragment derselben; sowie
(ii) Nukleinsäuren, die mit einer Nukleinsäure nach (i) spezifisch hybridisieren;
(iii) Nukleinsäuren, die mindestens 80 % identisch mit der Nukleinsäure nach (i) oder (ii) sind, sowie
(iv) Nukleinsäuren, die komplementär mit einer Nukleinsäure nach (i) bis (iii) sind; und
eine weitere Sonde die mit einem konservierten Bereich der Plasmide pPLa, pFRA oder pYV *aus Yersinia pestis* oder eines Teils derselben hybridisiert und aus der Gruppe ausgewählt ist aus:
(i) einer Nukleinsäure, die eine Nukleinsäuresequenz aus SEQ ID NO: 11 oder 12 umfassen oder ein 15 bis 20 Nukleotide langes Fragment derselben;
(ii) Nukleinsäuren, die mit einer Nukleinsäure nach (i) spezifisch hybridisieren;
(iii) einer Nukleinsäure, die mindestens 80 % identisch er Nukleinsäure nach (i) oder (ii) ist;
(iv) Nukleinsäuren, die komplementär mit einer Nukleinsäure nach (i) bis (iii) sind.

15. Kit nach Anspruch 14 enthaltend zusätzlich eine Amplifikationskontrolle für die Amplifikationsreaktion.

16. Kit nach Anspruch 15, wobei die Nukleinsäure zur Kontrolle der Amplifikation ausgewählt ist aus:
(i) einer Nukleinsäure, die eine Nukleinsäuresequenz nach SEQ ID NO: 13 umfasst oder ein 15 bis 19 Nukleotide langes Fragment derselben;
(ii) einer Nukleinsäure, die mit einer Nukleinsäure nach (i) spezifisch hybridisiert;
(iii) einer Nukleinsäure, die mindestens 80 % identisch mit einer Nukleinsäure nach (i) oder (ii) ist.

17. Kit gemäß einem der Ansprüche 14 -16, **dadurch gekennzeichnet, dass** die Nukleinsäuren ausgewählt sind aus DNA, RNA oder PNA.

18. Kit gemäß einem der Ansprüche 14-16, **dadurch gekennzeichnet, dass** die Nukleinsäure **dadurch** modifiziert ist, dass bis zu 20 % der Nukleotide, mindestens jedoch 1 Nukleotid in 10 aufeinander folgende Nukleotide durch Nukleotide ersetzt sind, die in Bakterien nicht natürlich vorkommen.

19. Kit gemäß einem der Ansprüche 14-18. **dadurch gekennzeichnet, dass** in die Nukleinsäuren zur Erzeugung eines direkt oder indirekt nachweisbaren Signals eine Modifikation eingeführt wird, wobei die Modifikation bestehen kann aus:
(i) einer radioaktiven Markierung
(ii) farbigen Gruppen,
(iii) fluoreszierenden Gruppen,
(iv) chemolumineszierenden Gruppen,
(v) Gruppen zur Immobilisierung an einer festen Phase und/oder
(vi) Gruppen, die eine indirekte oder direkte Nachweisreaktion, mit Hilfe von Antikörpern, Antigenen, Enzymen und/oder Substanzen mit Affinität zu Enzymen oder Enzymkomplexen erlauben, oder eine Kombination von Modifikationen gemäß zwei oder mehr der unter (i) bis (vi) genannten Modifikationen.

20. Verwendung eines Kits gemäß einem der Ansprüche 14 - 19, zum Nachweisen von Mikroorganismen der Spezies *Yersinia pestis*/ *Yersinia* pseudotuberculosis und/oder der Differenzierung zwischen *Yersinia pestis* und *Yersinia pseudotuberculosis.*

21. Verwendung eines Kits nach einem der Ansprüche 14 - 19 in einem Verfahren nach einem der Ansprüche 1-13.

## Claims

1. A method for the detection of microorganisms of the species *Yersinia pestis* and *Yersinia pseudotuberculosis* and for differentiation between *Yersinia pestis* and *Yersinia pseudotuberculosis* in a sample, the method comprising the following steps:
(a) bringing the sample into contact with a combination of at least two primer pairs, which hybridise with an area of their microbial genome preserved in the species *Yersinia pestis* / *Yersinia pseudotuberculosis* or a part thereof, a first primer pair hybridising with a preserved area of a bacterial 16S rDNA gene or a part thereof and a second primer pair hybridising with a preserved area of the plasmids pPla, pFra or pYV from *Yersinia pestis* or a part thereof;
(b) amplification of the microbial genome or a part thereof to generate at least two amplification fragments;
(c) bringing the amplification fragments obtained in step (b) into contact with at least one probe specific for *Yersinia pestis* and *Yersinia pseudotuberculosis,* which hybridises with a preserved area from the bacterial 16S rDNA genes or a part thereof, and hybridises with the amplification fragments with formation of hybrid nucleic acids, and a probe which hybridises with a preserved area of the plasmids pPla, pFra or pYV from *Yersinia pestis* or a part thereof;
(d) detection of the hybrid nucleic acids.

2. The method according to Claim 1, **characterised in that** the at least two primer pairs are chosen from the following group of nucleic acids:
(i) a nucleic acid that contains a nucleic acid sequence according to SEQ ID NO. 1-9 or a 15 to 25 nucleotide long fragment of the same;
(ii) a nucleic acid which hybridises specifically with a nucleic acid according to (i);
(iii) a nucleic acid which is at least 70% identical with a nucleic acid according to (i) or (ii);
(iv) a nucleic acid which is complementary to a nucleic acid according to (i) to (iii);

3. The method according to Claim 1, **characterised in that** one or more nucleic acids are used as the probe, which are chosen from the following group:
(i) a nucleic acid that contains a nucleic acid sequence according to SEQ ID NO. 10-12 or a 15 to 20 nucleotide long fragment of the same;
(ii) a nucleic acid which hybridises specifically with a nucleic acid according to (i);
(iii) a nucleic acid which is at least 80% identical with a nucleic acid according to (i) or (ii);
(iv) a nucleic acid which is complementary to a nucleic acid according to (i) to (iii).

4. The method according to one of the Claims 1 to 3, **characterised in that** the nucleic acid is a DNA, RNA or PNA.

5. The method according to one of the Claims 1 to 4, **characterised in that** up to 20% of the nucleotides, but at least 1 nucleotide in 10 consecutive nucleotides is replaced by nucleotides that do not occur naturally in bacteria.

6. The method according to one of the Claims 1 to 5, **characterised in that** a modification is introduced into the nucleic acid for production of a signal that can be detected directly or indirectly, wherein the modification may consist of (i) a radioactive marking, (ii) coloured groups, (iii) fluorescent groups, (iv) chemoluminescent groups, (v) groups for immobilisation at a solid phase and / or (vi) groups which permit an indirect or direct detection reaction using antibodies, antigens, enzymes and / or substances with an affinity to enzymes or enzyme complexes, or a combination of modifications according to two or more of the modifications listed under (i) to (vi).

7. The method according to one of the Claims 1 to 6, **characterised in that** the amplification includes a polymerase chain reaction (PCR).

8. The method according to Claim 7, **characterised in that** the polymerase chain reaction (PCR) comprises a multiplex PCR.

9. The method according to Claim 7, **characterised in that** the polymerase chain reaction (PCR) comprises an online or real-time PCR.

10. The method according to one of the Claims 1 to 6, **characterised in that** the amplification comprises a ligase chain reaction, an isothermic nucleic acid amplification or a Qβ replication.

11. The method according to one of the Claims 1 to 10, **characterised in that** an amplification control is carried out for the amplification.

12. The method according to Claim 11, **characterised in that** the amplification control contains a DNA sequence of maximum 1000 nucleotides.

13. The method according to Claim 12, **characterised in that** the DNA sequence of the amplification control is selected from the following group:
(i) a nucleic acid that contains a nucleic acid sequence according to SEQ ID NO. 13 or a 15 to 19 nucleotide long fragment of the same;
(ii) a nucleic acid which hybridises specifically with a nucleic acid according to (i);
(iii) a nucleic acid which is at least 80% identical with a nucleic acid according to (i) or (ii);
(iv) a nucleic acid which is complementary to a nucleic acid according to (i) to (ii).

14. A kit for the detection of microorganisms of the species *Yersinia pestis* / *Yersinia pseudotuberculosis* and for differentiation between *Yersinia pestis* and *Yersinia pseudotuberculosis,* containing
a) two primer pairs, wherein
a first primer pair hybridises with a preserved area of a bacterial 16S rDNA gene or a part thereof and is selected form the group comprising:
(i) nucleic acids that contain a nucleic acid sequence according to SEQ ID NO. 5-9 or a 15 to 25 nucleotide long fragment of the same;
(ii) nucleic acids which hybridise specifically with a nucleic acid according to (i);
(iii) nucleic acids which are at least 70% identical with a nucleic acid according to (i) or (ii);
(iv) nucleic acids which are complementary to a nucleic acid according to (i) to (iii),
wherein the used combination is selected from the primers such that they can be used in an amplification reaction, i.e. one nucleic acid hybridises with a first preserved area of the first strand of the target DNA and the other nucleic acid to a second preserved area of the DNA strand complementary to the strand, wherein the desired target area of the DNA is enclosed; and a second primer pair which hybridises with a preserved area of the plasmids pPLa, pFra or PYV from *Yersinia pestis* or a part thereof and is selected from
(i) nucleic acids that contain a nucleic acid sequence according to SEQ ID NO. 1-4 or a 15 to 25 nucleotide long fragment of the same; and
(ii) nucleic acids which hybridise specifically with a nucleic acid according to (i);
(iii) nucleic acids which are at least 70% identical with the nucleic acid according to (i) or (ii); and
(iv) nucleic acids which are complementary to a nucleic acid according to (i) to (iii); and
b) two probes, wherein
one probe hybridises with a preserved area from the bacterial 16S rDNA genes or a part thereof and is
(i) selected from nuclei0c acids that contain a nucleic acid sequence from SEQ ID NO. 10, or a 15 to 25 nucleotide long fragment of the same; and
(ii) nucleic acids which hybridise specifically with a nucleic acid according to (i);
(iii) nucleic acids which are at least 80% identical with a nucleic acid according to (i) or (ii); and
(iv) nucleic acids which are complementary to a nucleic acid according to (i) to (iii); and
a further probe hybridised with a preserved area of the plasmids pPLA, pFRA or pYV from *Yersinia pestis* or a part thereof and selected from the group of:
(i) a nucleic acid that contains a nucleic acid sequence from SEQ ID NO. 11 or 12 or a 15 to 20 nucleotide long frag ment of the same;
(ii) nucleic acids which hybridise specifically with a nucleic acid according to (i);
(iii) a nucleic acid which is at least 80% identical with a nucleic acid according to (i) or (ii);
(iv) nucleic acids which are complementary to a nucleic acid according to (i) to (iii).

15. The kit according to Claim 14, additionally containing an amplification control for the amplification reaction.

16. The kit according to Claim 15, wherein the nucleic acid for the control of the amplification is selected from:
(i) a nucleic acid that contains a nucleic acid sequence according to SEQ ID NO. 13 or a 15 to 19 nucleotide long fragment of the same;
(ii) a nucleic acid which hybridises specifically with a nucleic acid according to (i);
(iii) a nucleic acid which is at least 80% identical with a nucleic acid according to (i) or (ii).

17. The kit according to one of the Claims 14 to 16, **characterised in that** the nucleic acids are selected from DNA, RNA or PNA.

18. The kit according according to one of the Claims 14 to 16, **characterised in that** the nucleic acid is modified **in that** up to 20% of the nucleotides, but at least 1 nucleotide in 10 consecutive nucleotides is replaced by nucleotides that do not occur naturally in bacteria.

19. The kit according to one of the Claims 14 to 18, **characterised in that** a modification is introduced into the nucleic acids for production of a signal that can be detected directly or indirectly, wherein the modification may consist of
(i) a radioactive marking,
(ii) coloured groups,
(iii) fluorescent groups,
(iv) chemoluminescent groups,
(v) groups for immobilisation at a solid phase and / or
(vi) groups which permit an indirect or direct detection reaction using antibodies, antigens, enzymes and / or substances with an affinity to enzymes or enzyme complexes, or a combination of modifications according to two or more of the modifications listed under (i) to (vi).

20. Use of a kit according to one of the Claims 14 to 19, for the detection of microorganisms of the species *Yersinia pestis* / *Yersinia pseudotuberculosis* and / or for differentiation between *Yersinia pestis* and *Yersinia pseudotuberculosis.*

21. Use of a kit according to one of the Claims 14 to 19 in a method according to one of the Claims 1 to 13.

## Revendications

1. Procédé de détection de micro-organismes de l'espèce *Yersinia pestis* et *Yersinia pseudotuberculosis* et de différenciation entre *Yersinia pestis* et *Yersinia pseudotuberculosis* dans un échantillon, où le procédé comprend les étapes suivantes :
(a) la mise en contact de l'échantillon avec une combinaison d'au moins deux paires d'amorces, qui s'hybrident avec un domaine de son génome microbien ou une partie de celui-ci conservé dans l'espèce *Yersinia pestis* et *Yersinia pseudotuberculosis,* où une première paire d'amorces s'hybride avec un domaine conservé d'un gène d'ADNr 16S bactérien ou une partie de celui-ci et une deuxième paire d'amorces s'hybride avec un domaine conservé des plasmides pPla, pFra ou pYV provenant de *Yersinia pestis* ou une partie de celui-ci;
(b) amplification des génomes microbiens ou d'une partie de ceux-ci pour produire au moins deux fragments d'amplification;
(c) la mise en contact des fragments d'amplification obtenus à l'étape (b) avec au moins une sonde spécifique pour *Yersinia pestis* et *Yersinia pseudotuberculosis,* qui s'hybride avec un domaine conservé provenant des gènes d'ADNr 16S bactérien ou une partie de ceux-ci, et avec les fragments d'amplification en formant des acides nucléiques hybrides ; et une sonde, qui s'hybride avec un domaine conservé des plasmides pPla, pFra ou pYV provenant de *Yersinia pestis* ou d'une partie de celui-ci;
(d) détection des acides nucléiques hybrides.

2. Procédé selon la revendication 1, **caractérisé en ce que** les au moins deux paires d'amorces sont choisies dans le groupe suivant d'acides nucléiques .
(i) un acide nucléique, qui comprend une séquence d'acide nucléique d'après la SEQ ID N°1 - 9 ou un fragment long de 15 à 25 nucléotides de celle-ci ;
(ii) un acide nucléique qui s'hybride spécifiquement avec un acide nucléique selon (i);
(iii) un acide nucléique qui est identique à au moins 70 % avec un acide nucléique selon (i) ou (ii) ;
(iv) un acide nucléique qui est complémentaire à un acide nucléique selon (i) à (iii).

3. Procédé selon la revendication 1, **caractérisé en ce que** l'on utilise comme sonde un ou plusieurs acides nucléiques, qui sont choisis dans le groupe suivant:
(i) un acide nucléique, qui comprend une séquence d'acide nucléique d'après la SEQ ID N°10 - 12 ou un fragment long de 15 à 20 nucléotides de celle-ci ;
(ii) un acide nucléique qui s'hybride spécifiquement avec un acide nucléique selon (i) ;
(iii) un acide nucléique qui est identique à au moins 80 % avec un acide nucléique selon (i) ou (ii) ;
(iv) un acide nucléique qui est complémentaire à un acide nucléique selon (i) à (iii).

4. Procédé selon l'une des revendications 1 à 3, **caractérisé en ce que** l'acide nucléique est un ADN, ARN ou APN.

5. Procédé selon l'une des revendications 1 à 4, **caractérisé en ce que** jusqu'à 20 % des nucléotides, soit au moins 1 nucléotide sur 10 nucléotides successifs est remplacé par des nucléotides qui ne se trouvent pas naturellement dans des bactéries.

6. Procédé selon l'une des revendications 1 à 5, **caractérisé en ce que** l'on introduit une modification dans l'acide nucléique pour produire un signal directement ou indirectement détectable, où la modification peut se composer (i) d'un marquage radioactif, (ii) de groupes colorés, (iii) de groupes fluorescents, (iv) de groupes chimioluminescents, (v) de groupes pour l'immobilisation sur une phase solide et/ou (vi) de groupes qui permettent une réaction de détection directe ou indirecte, à l'aide d'anticorps, d'antigènes, d'enzymes et/ou de substances ayant une affinité pour des enzymes ou complexes enzymatiques, ou une combinaison de modifications selon deux ou plus des modifications citées ci-dessus au (i) à (vi).

7. Procédé selon l'une des revendications 1 à 6, **caractérisé en ce que** l'amplification comprend une réaction en chaîne polymérase (PCR).

8. Procédé selon la revendication 7, **caractérisé en ce que** la réaction en chaîne polymérase (PCR) comprend une PCR multiplexe.

9. Procédé selon la revendication 7, **caractérisé en ce que** la réaction en chaîne polymérase (PCR) comprend une PCR en ligne ou en temps réel.

10. Procédé selon l'une des revendications 1 à 6, **caractérisé en ce que** l'amplification comprend une réaction en chaîne ligase, une amplification d'acide nucléique isotherme ou une réplication Qβ.

11. Procédé selon l'une des revendications 1 à 10, **caractérisé en ce que** l'on réalise lors de l'amplification un contrôle d'amplification.

12. Procédé selon la revendication 11,
**caractérisé en ce que** le contrôle d'amplification comprend une séquence d'ADN de 1000 nucléotides au maximum.

13. Procédé selon la revendication 12,
**caractérisé en ce que** la séquence d'ADN du contrôle d'amplification est choisie dans le groupe suivant :
(i) un acide nucléique, qui comprend une séquence d'acide nucléique d'après la SEQ ID N°13 ou un fragment long de 15 à 19 nucléotides de celle-ci ;
(ii) un acide nucléique qui s'hybride spécifiquement avec un acide nucléique selon (i) ;
(iii) un acide nucléique qui est identique à au moins 80 % avec un acide nucléique selon (i) ou (ii) ;
(iv) un acide nucléique qui est complémentaire à un acide nucléique selon (i) à (iii).

14. Kit de détection de micro-organismes de l'espèce de l'espèce *Yersinia pestis* / *Yersinia pseudotuberculosis* et de différenciation entre *Yersinia pestis* et *Yersinia pseudotuberculosis,* contenant
a) deux paires d'amorces, où
une première paire d'amorces s'hybride avec un domaine conservé d'un gène d'ADNr 16S bactérien ou une partie de celui-ci et est choisie dans le groupe comprenant
(i) des acides nucléiques, qui comprennent une séquence d'acide nucléique d'après la SEQ ID N°5 à 9 ou un fragment long de 15 à 25 nucléotides de celle-ci ;
(ii) des acides nucléiques, qui s'hybrident spécifiquement avec un acide nucléique selon (i);
(iii) des acides nucléiques, qui sont identiques à au moins 70 % avec l'acide nucléique selon (i) ou (ii) ; et
(iv) des acides nucléiques, qui sont complémentaires à un acide nucléique selon (i) à (iii),
où la combinaison d'amorces mise en oeuvre est choisie de manière à pouvoir être mise en oeuvre dans une réaction d'amplification, ce qui veut dire qu'un acide nucléique s'hybride avec un premier domaine conservé du premier brin de l'ADN cible et l'autre acide nucléique sur un deuxième domaine conservé du brin d'ADN complémentaire au premier brin, où le domaine cible voulu de l'ADN est inclus ; et
une deuxième paire d'amorces s'hybride avec un domaine conservé des plasmides pPla, pFra ou pYV provenant de *Yersinia pestis* ou une partie de celui-ci et est choisie parmi:
(i) des acides nucléiques, qui comprennent une séquence d'acide nucléique d'après la SEQ ID N°1 à 4 ou un fragment long de 15 à 25 nucléotides de celle-ci;
(ii) des acides nucléiques, qui s'hybrident spécifiquement avec un acide nucléique selon (i) ;
(iii) des acides nucléiques, qui sont identiques à au moins 70 % avec l'acide nucléique selon (i) ou (ii) ; et
(iv) des acides nucléiques, qui sont complémentaires à un acide nucléique selon (i) à (iii) ; et
b) deux sondes, où
une sonde s'hybride avec un domaine conservé des gènes d'ADNr 16S bactériens ou une partie de celui-ci et est choisie dans le groupe comprenant
(i) des acides nucléiques, qui comprennent une séquence d'acide nucléique d'après la SEQ ID N°10 ou un fragment long de 15 à 20 nucléotides de celle-ci ;
(ii) des acides nucléiques, qui s'hybrident spécifiquement avec un acide nucléique selon (i) ;
(iii) des acides nucléiques, qui sont identiques à au moins 80 % avec l'acide nucléique selon (i) ou (ii), et
(iv) des acides nucléiques, qui sont complémentaires à un acide nucléique selon (i) à (iii) ; et
une deuxième sonde s'hybride avec un domaine conservé des plasmides pPla, pFra ou pYV provenant de *Yersinia pestis* ou une partie de celui-ci et est choisie parmi:
(i) des acides nucléiques, qui comprennent une séquence d'acide nucléique d'après la SEQ ID N°11 ou 12 ou un fragment long de 15 à 20 nucléotides de celle-ci ;
(ii) des acides nucléiques, qui s'hybrident spécifiquement avec un acide nucléique selon (i) ;
(iii) des acides nucléiques, qui sont identiques à au moins 80 % avec l'acide nucléique selon (i) ou (ii) ; et
(iv) des acides nucléiques, qui sont complémentaires à un acide nucléique selon (i) à (iii).

15. Kit selon la revendication 14 contenant en plus un contrôle d'amplification pour la réaction d'amplification.

16. Kit selon la revendication 15, où l'acide nucléique pour le contrôle de l'amplification est choisi parmi .
(i) un acide nucléique, qui comprend une séquence d'acide nucléique d'après la SEQ ID N°13 ou un fragment long de 15 à 19 nucléotides de celle-ci ;
(ii) un acide nucléique qui s'hybride spécifiquement avec un acide nucléique selon (i) ;
(iii) un acide nucléique qui est identique à au moins 80 % avec un acide nucléique selon (i) ou (ii).

17. Kit selon l'une des revendications 14 à 16, **caractérisé en ce que** les acides nucléiques sont choisis parmi l'ADN, l'ARN ou l'APN.

18. Kit selon l'une des revendications 14 à 16, **caractérisé en ce que** l'acide nucléique est modifié **en ce que** jusqu'à 20 % des nucléotides, soit au moins 1 nucléotide sur 10 nucléotides successifs, sont remplacés par des nucléotides qui ne se trouvent pas naturellement dans des bactéries.

19. Kit selon l'une des revendications 14 à 18, **caractérisé en ce que** l'on introduit une modification dans les acides nucléiques pour produire un signal directement ou indirectement détectable, où la modification peut se composer
(i) d'un marquage radioactif,
(ii) de groupes colorés,
(iii) de groupes fluorescents,
(iv) de groupes chimioluminescents,
(v) de groupes pour l'immobilisation sur une phase solide et/ou
(vi) de groupes qui permettent une réaction de détection directe ou indirecte, à l'aide d'anticorps, d'antigènes, d'enzymes et/ou de substances ayant une affinité pour des enzymes ou complexes enzymatiques, ou une combinaison de modifications selon deux ou plus des modifications citées ci-dessus au (i) à (vi).

20. Utilisation d'un kit selon l'une des revendications 14 à 19, pour la détection de micro-organismes de l'espèce *Yersinia pestis* / *Yersinia pseudotuberculosis* et de différenciation entre *Yersinia pestis* et *Yersinia pseudotuberculosis.*

21. Utilisation d'un kit selon l'une des revendications 14 à 19 dans un procédé selon l'une des revendications 1 à 13.


### SEQUENZ PROTOKOLL

<110> Biotecon Diagnostics GmbH
<120> Nukleinsäuremolekülsatz für Yersinia-Nachweis
<130> BCD-2001-yp
<140>
   <141>
<160> 13
<170> PatentIn Ver. 2.1
<210> 1
   <211> 20
   <212> DNA
   <213> Yersinia sp.
<400> 1
   atcttacttt ccgtgagaag 20
<210> 2
   <211> 20
   <212> DNA
   <213> *Yersinia sp.*
<400> 2
   cttggatgtt gagcttccta 20
<210> 3
   <211> 24
   <212> DNA
   <213> Yersinia sp.
<400> 3
   ggattattgg ttagatacgg ttac 24
<210> 4
   <211> 22
   <212> DNA
   <213> Yersinia sp.
<400> 4
   ggtgatccca tgtacttaac at 22
<210> 5
   <211> 16
   <212> DNA
   <213> Yersinia sp.
<400> 5
   ggcagagatg ctaaag 16
<210> 6
   <211> 20
   <212> DNA
<400> 6
   atttggcaga gatgctaaag 20
<210> 7
   <211> 26
   <212> DNA
   <213> *Yersinia sp.*
<400> 7
   ctcccatggt gtgacgggcg gtgtgt 26
<210> 8
   <211> 17
   <212> DNA
   <213> Yersinia sp.
<400> 8
   tgtgacgggc ggtgtgt 17
<210> 9
   <211> 22
   <212> DNA
   <213> Yersinia sp.
<400> 9
   ctacttcttt tgcaacccac tc 22
<210> 10
   <211> 21
   <212> DNA
   <213> Yersinia sp.
<400> 10
   agtcatcatg gcccttacga g 21
<210> 11
   <211> 20
   <212> DNA
   <213> Yersinia sp.
<400> 11
   gatgacgtcg tcttggctac 20
<210> 12
   <211> 20
   <212> DNA
   <213> Yersinia sp.
<400> 12
   ggtctgcaat atcgcttctg 20
<210> 13
   <211> 20
   <212> DNA
<213> Künstliche Sequenz
   <220>
   <223> Beschreibung der künstlichen Sequenz: ST-Sonde
<400> 13
   gactacggaa ttccgctgtc 20